# EUROPEAN PATENT SPECIFICATION

(11) **EP 1 900 348 B1**
(45) Date of publication and mention of the grant of the patent: **28.11.2012**
(21) Application number: 07253576.8
(22) Date of filing: 10.09.2007
(51) Int. Cl.: A61F 13/00, A61F 13/02

(54) **Thin film dressing**
Dünnfilmverband
Pansement à film mince

(30) Priority: 12.09.2006 US 844008 P
(43) Date of publication of application: 19.03.2008
(73) Proprietor: Tyco Healthcare Group LP, Mansfield, MA 02048 (US)
(72) Inventor: Vitaris, Ronald F., Worcester, Massachusetts 01606 (US)
(74) Representative: Jones, Nicholas Andrew

(56) References cited:
- DE-A1- 10 345 665
- US-A- 4 341 207
- US-A- 4 649 909
- US-A- 5 593 395
- US-A- 5 733 251
- US-A- 6 149 614
- US-B1- 6 277 401

## Description

### TECHNICAL FIELD

The present disclosure relates to wound dressings. The dressings may include thin films having custom levels of adhesion and moisture vapor transmission rate ("MVTR").

### BACKGROUND OF RELATED ART

The use of self adherent wound dressings is known. Such dressings may have various configurations, including windows therein to permit viewing of a wound site. For example, U.S. Patent Nos. 6,124,520, 6,124,521, and 6,841,715 all disclose dressings possessing a window permitting the visualization of a wound site while the dressing is in place. Other dressings possessing windows include, for example, those disclosed in U.S. Patent Nos. 5,531,855, 5,738,642, 6,169,224, and 6,685,682.

Despite the wide variety of known dressings and configurations thereof, there still remains a need for dressings which possess desirable adherence properties and moisture transmission characteristics.

### SUMMARY

The present disclosure provides articles which are used as wound dressings or bandages, and methods for their manufacture. In embodiments, an article of the present disclosure includes at least one hydrophilic layer having a skin-facing side and a side opposite thereto, at least one hydrophobic layer adjacent to the hydrophilic layer on the side opposite the skin-facing side, a delivery layer adjacent to the hydrophobic layer on the side opposite to the hydrophilic layer, and a release layer on the side of the hydrophilic layer opposite to the hydrophobic layer, wherein the hydrophobic layer possesses a window therein formed by the removal of a central portion of the hydrophobic layer. Articles of the present disclosure may possess a thickness from substantially 0.076mm (3 mils) to substantially 0.279mm (11 mils).

In embodiments, the hydrophilic layer may have a moisture vapour transition rate from substantially 400 gr/m²/24-hour to substantially 3000 gr/m²/24-hour and a thickness from substantially 0.0102mm (0.4 mils) to substantially 0.038mm (1.5 mils). In embodiments, the hydrophobic layer may have a moisture vapour transition rate from substantially 250 gr/m²/24-hour to substantially 1000 gr/m²/24-hour and a thickness from substantially 0.0102mm (0.4 mils) to substantially 0.127mm (5 mils).

The skin-facing side of the hydrophilic layer, the hydrophobic layer, or both, may possess a coating on at least a portion thereof comprising a medically accepted adhesive.

In some embodiments, the delivery layer may also possess a window therein formed by the removal of a central portion of the delivery layer.

Articles of the present disclosure may also possess an adhesive tape between the hydrophobic layer and the delivery layer, and/or a stabiliser layer between the hydrophilic layer and the hydrophobic layer, and/or an absorbent material on the skin-facing side of the hydrophilic layer.

In some embodiments, the hydrophilic layer, the hydrophobic layer, and the delivery layer may possess notches therein which are contiguous with each other. These notches may permit the placement of an article of the present disclosure around a needle or similar device to assist in keeping any catheter and/or intravenous line from moving excessively.

In embodiments, articles of the present disclosure include at least one hydrophilic layer possessing a moisture vapour transition rate from substantially 400 gr/m²/24-hour to substantially 3000 gr/m²/24-hour and having a skin-facing side, and a side opposite thereto, at least one hydrophobic layer adjacent to the hydrophilic layer on the side opposite the skin-facing side possessing a moisture vapour transition rate from substantially 250 gr/m²/24-hour to substantially 1000 gr/m²/24-hour and having a window therein formed by the removal of a central portion of the hydrophobic layer, a delivery layer adjacent to the hydrophobic layer on the side opposite the hydrophilic layer having a window therein formed by the removal of a central portion of the delivery layer, and a release layer on the side of the hydrophilic layer opposite to the hydrophobic layer, wherein the windows in the hydrophobic layer and the delivery layer are contiguous with each other, and the article has a thickness from substantially 0.076mm (3 mils) to substantially 0.279mm (11 mils).

In embodiments, articles of the present disclosure may include medicinal agents such as antimicrobials, analgesics, antipyretics, anesthetics, antiepileptics, antihistamines, anti-inflammatories, diagnostic agents, sympathomimetics, parasympathomimetics, cholinomimetics, antimuscarinics, antispasmodics, hormones, hormone analogs, growth factors, muscle relaxants, antineoplastics, immunosuppressants, steroids, polysaccharides, enzymes, tranquilizers, sulfonamides, vaccines, vitamins, antimalarials, anti-migraine agents, anti-parkinson agents, anticholinergics, cardiovascular agents, alkaloids, narcotics, opioid receptor antagonists, anti-cancer agents, anti-convulsants, anti-emetics, antihistamines, prostaglandins, cytotoxic drugs, estrogens, antibacterials, antifungals, antivirals, anticoagulants, anticonvulsants, antidepressants, immunological agents, antigens, blood coagulation factors, protein inhibitors, protein antagonists, protein agonists, nucleic acids, oligonucleotides, ribozymes, and combinations thereof.

### BRIEF DESCRIPTION OF THE DRAWINGS

Various embodiments of the present disclosure will be described herein below with reference to the figures wherein:

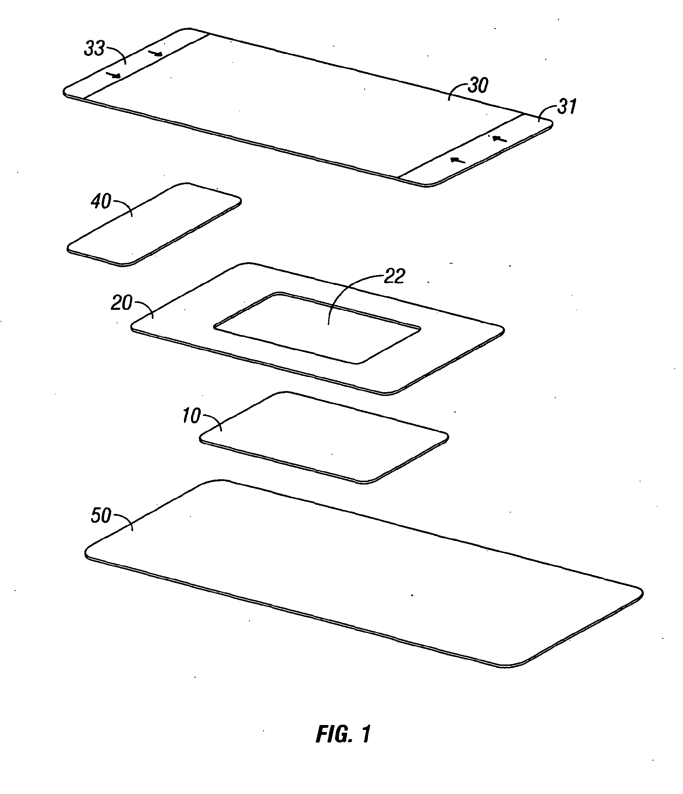

FIG. 1 is a depiction of a dressing of the present disclosure having a hydrophilic layer, a hydrophobic layer with a window therein, a delivery layer, an adhesive tape, and a release layer;

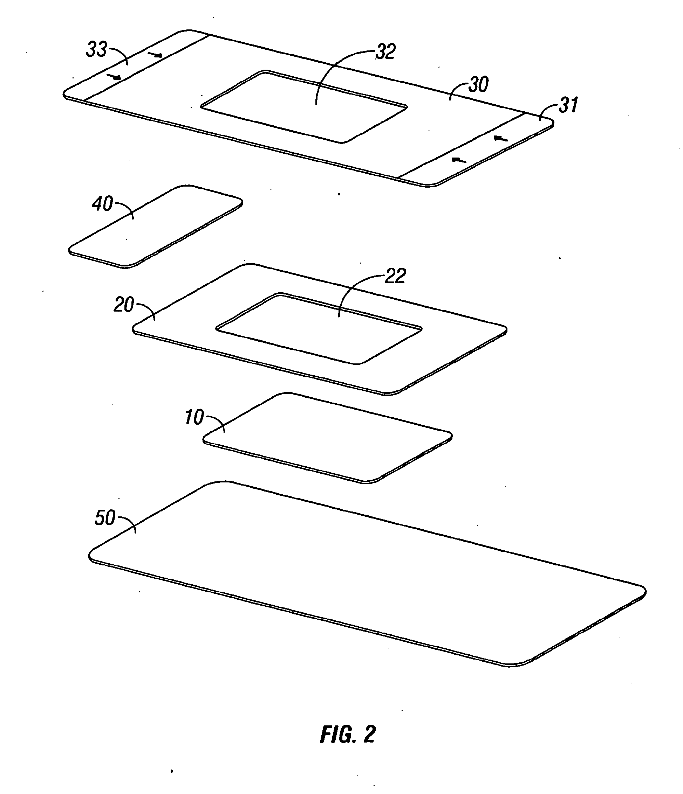

FIG. 2 is a depiction of an alternate dressing of the present disclosure having a hydrophilic layer, a hydrophobic layer with a window therein, a delivery layer with a window therein, an adhesive tape, and a release layer;

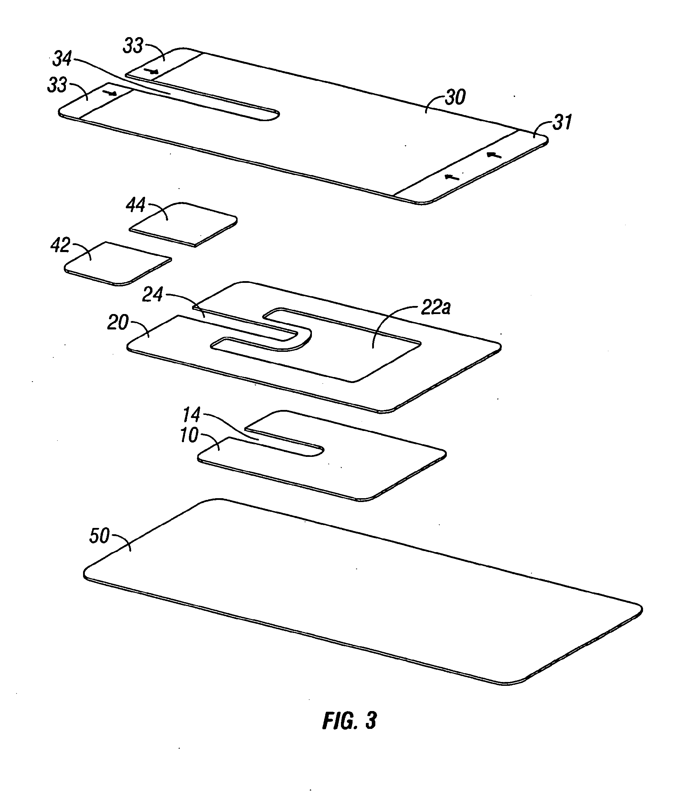

FIG. 3 is a depiction of an alternate dressing of the present disclosure having a hydrophilic layer with a notch therein, a hydrophobic layer with a window and a notch therein, a delivery layer with a notch therein, an adhesive tape in two pieces forming a notch, and a release layer;

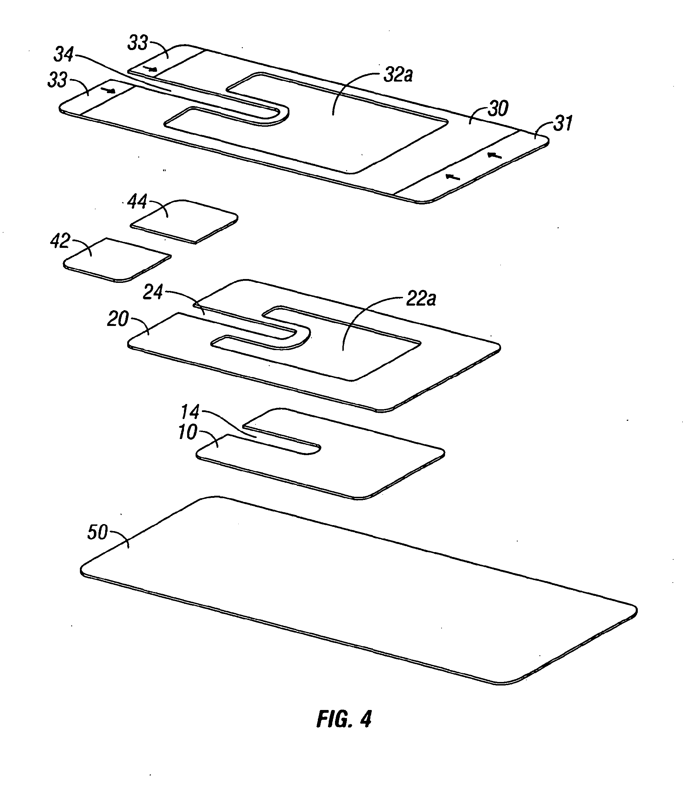

FIG. 4 is a depiction of an alternate dressing of the present disclosure having a hydrophilic layer with a notch therein, a hydrophobic layer with a window and a notch therein, a delivery layer with a window and a notch therein, an adhesive tape in two pieces forming a notch, and a release layer;

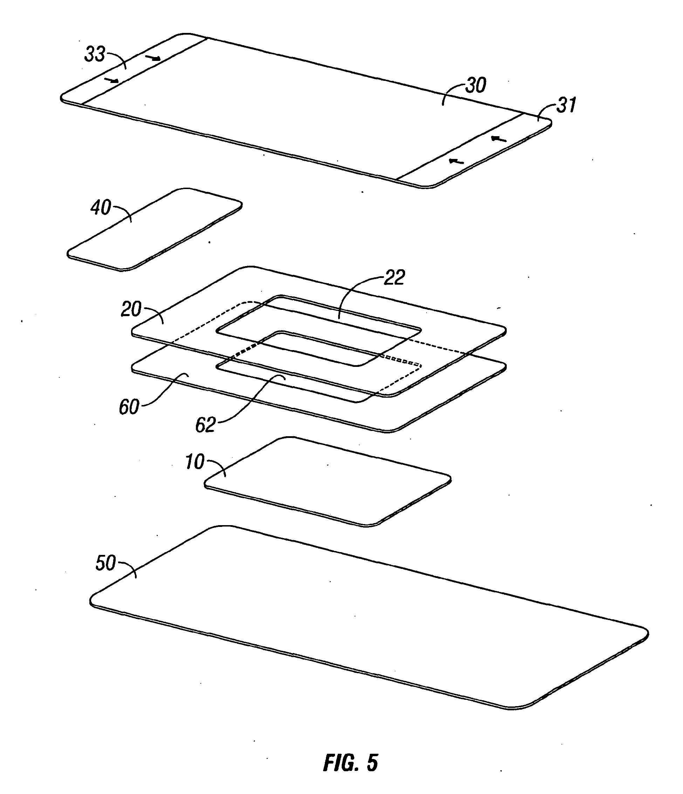

FIG. 5 is a depiction of an alternate dressing of the present disclosure possessing a hydrophilic layer, a hydrophobic layer with a window, a delivery layer, an adhesive tape, a release layer, and a stabilizer layer possessing a window therein located between the hydrophilic layer and the hydrophobic layer;

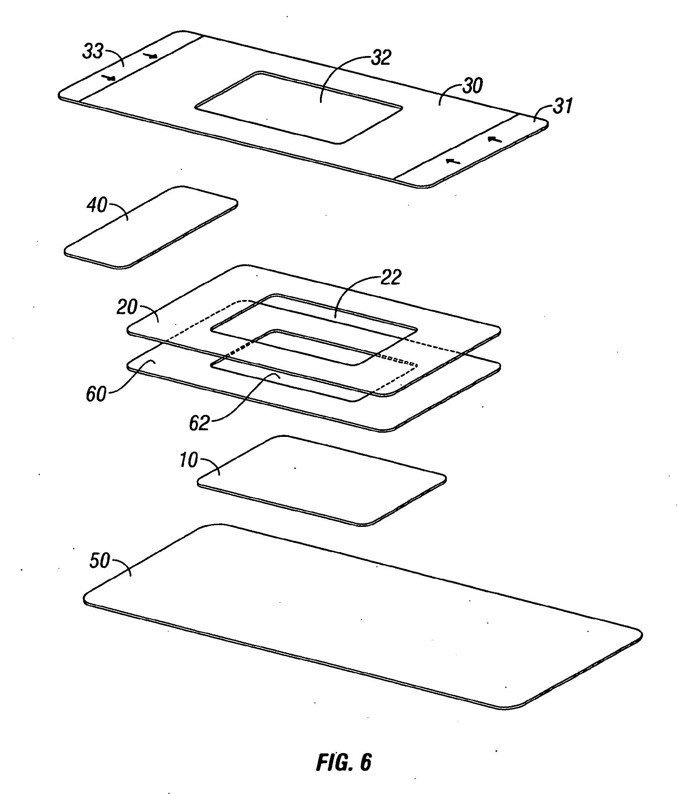

FIG. 6 is a depiction of an alternate dressing of the present disclosure having a hydrophilic layer, a hydrophobic layer with a window therein, a delivery layer with a window therein, an adhesive tape, a release layer, and a stabilizer layer possessing a window therein located between the hydrophilic layer and the hydrophobic layer;

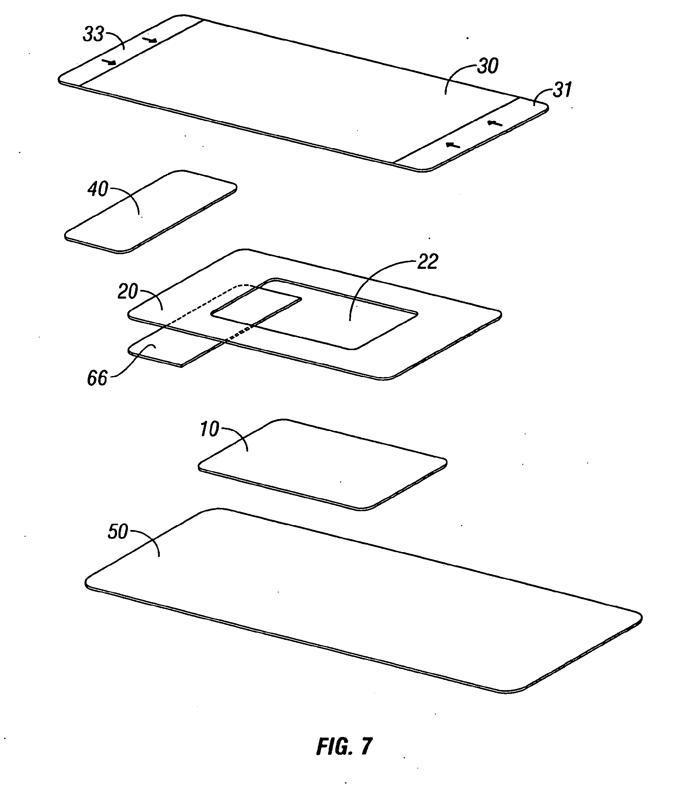

FIG. 7 is a depiction of an alternate dressing of the present disclosure having a hydrophilic layer, a hydrophobic layer with a window, a delivery layer, an adhesive tape, a release layer, and a stabilizer strip located between the hydrophilic layer and the hydrophobic layer on one edge of the hydrophilic layer and the hydrophobic layer;

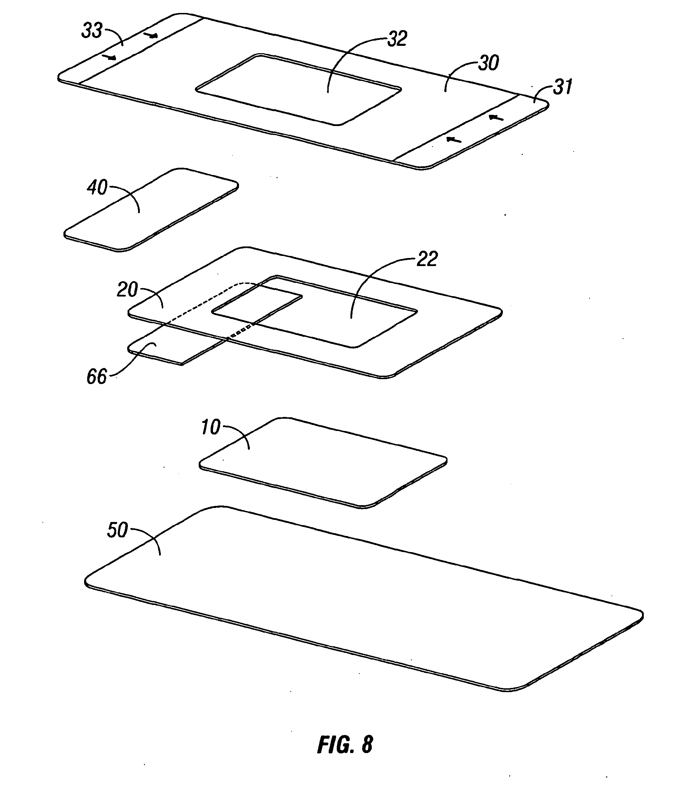

FIG. 8 is a depiction of an alternate dressing of the present disclosure having a hydrophilic layer, a hydrophobic layer with a window, a delivery layer with a window, an adhesive tape, a release layer, and a stabilizer strip located between the hydrophilic layer and the hydrophobic layer on one edge of the hydrophilic layer and the hydrophobic layer;

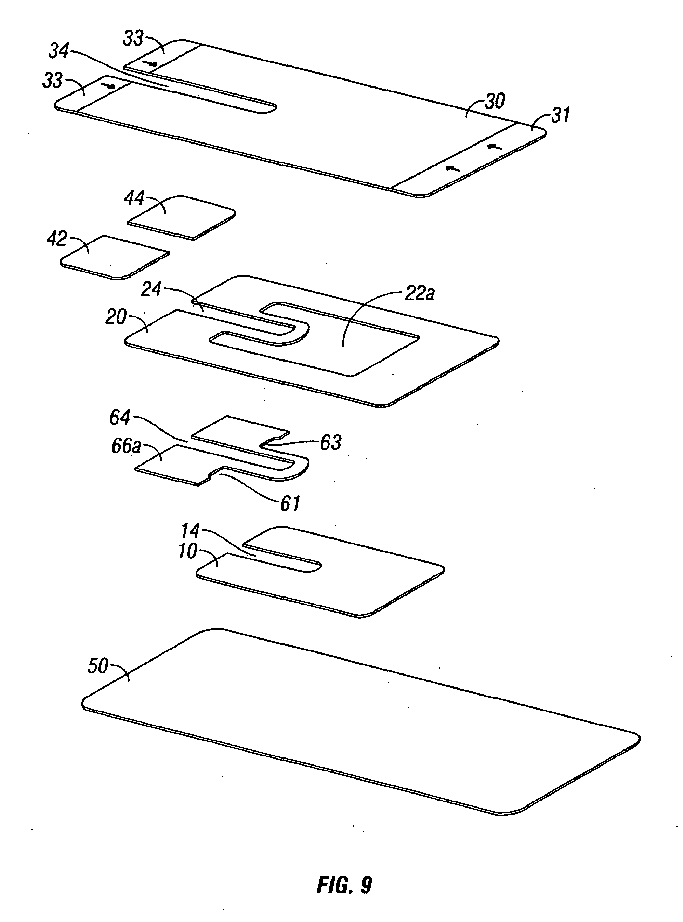

FIG. 9 is a depiction of an alternate dressing of the present disclosure having a hydrophilic layer having a notch, a hydrophobic layer with a window and a notch, a delivery layer with a notch, an adhesive tape in two pieces thereby forming a notch, a release layer, and a stabilizer strip possessing a notch therein and a sigmoidal configuration located between the hydrophilic layer and the hydrophobic layer on one edge of the hydrophilic layer and the hydrophobic layer;

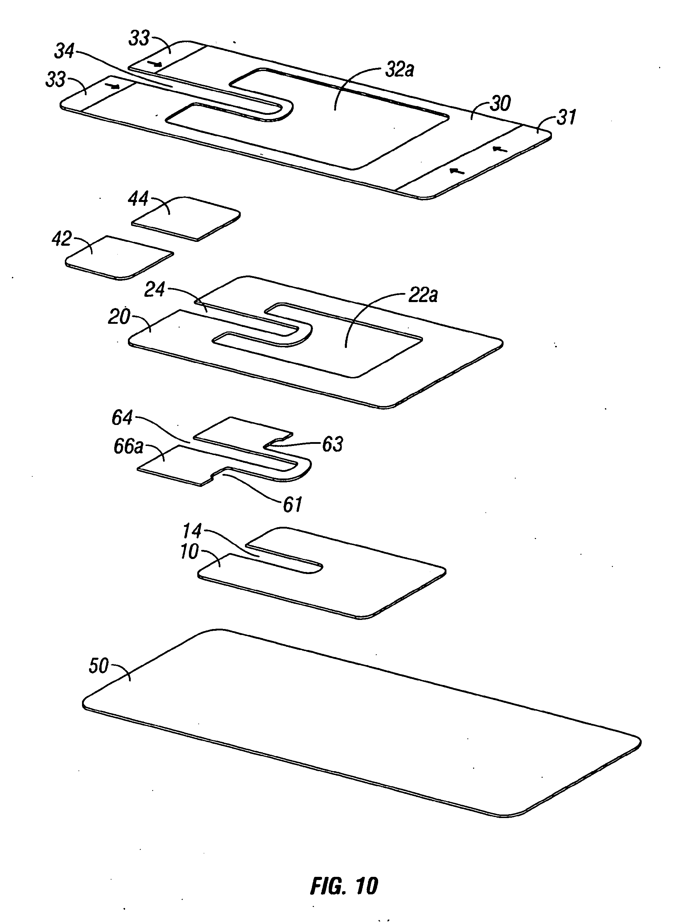

FIG. 10 is a depiction of an alternate dressing of the present disclosure having a hydrophilic layer having a notch, a hydrophobic layer with a window and a notch, a delivery layer having a window and a notch, an adhesive tape in two pieces thereby forming a notch, a release layer, and a stabilizer strip possessing a notch therein and a sigmoidal configuration located between the hydrophilic layer and the hydrophobic layer on one edge of the hydrophilic layer and the hydrophobic layer;

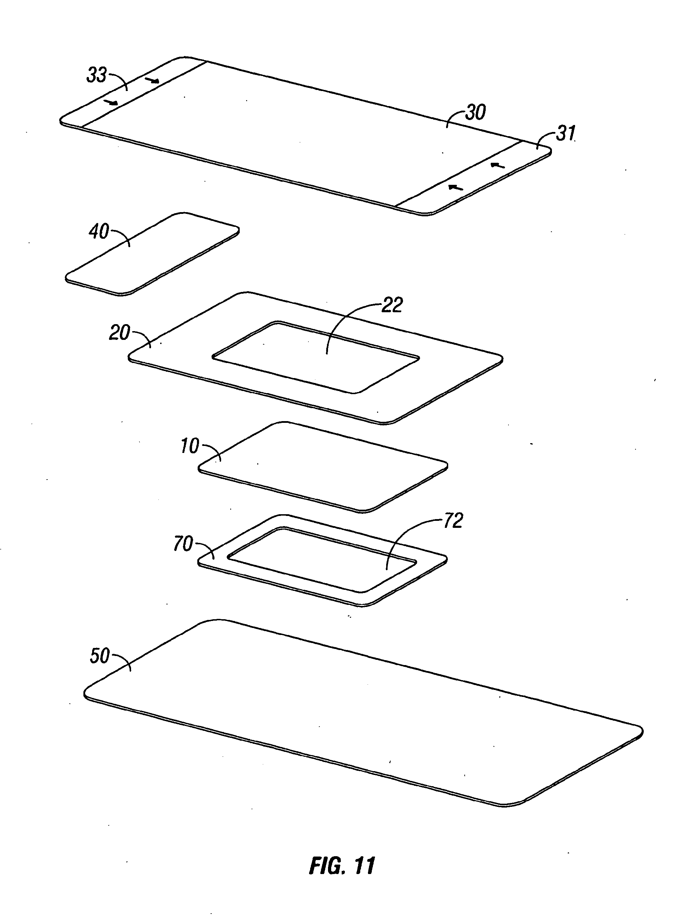

FIG. 11 is a depiction of an alternate dressing of the present disclosure having a hydrophilic layer, a hydrophobic layer with a window therein, a delivery layer, an adhesive tape, a release layer, and an absorbent layer with a window therein located between the hydrophilic layer and the release layer;

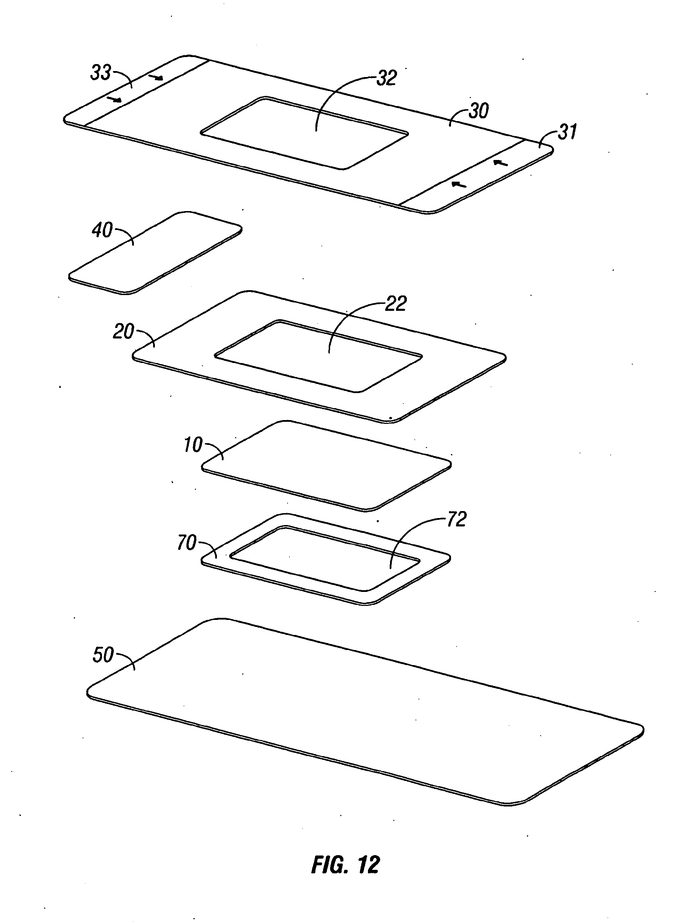

FIG. 12 is a depiction of an alternate dressing of the present disclosure having a hydrophilic layer, a hydrophobic layer with a window therein, a delivery layer with a window therein, an adhesive tape, a release layer, and an absorbent layer with a window therein located between the hydrophilic layer and the release layer; and

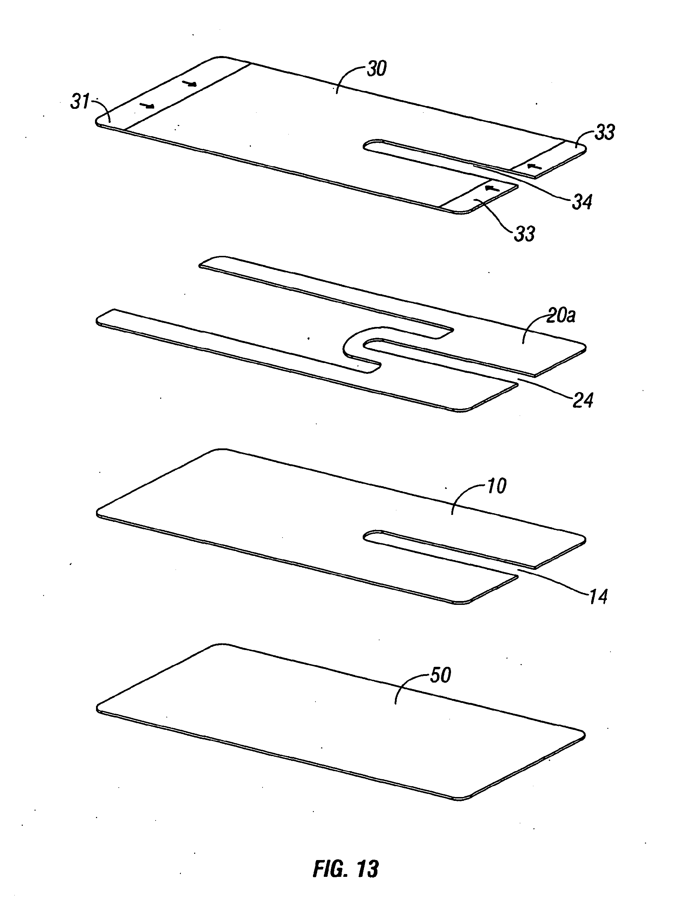

FIG. 13 is a depiction of an alternate dressing of the present disclosure having a hydrophilic layer with a notch, a hydrophobic layer at the periphery of the hydrophilic layer with a notch, a delivery layer with a notch, and a release layer.

### DETAILED DESCRIPTION

The dressings of the present disclosure possess various layers, in embodiments more than one layer. The dressings include a hydrophilic layer with a hydrophobic layer.

Suitable hydrophilic layers which may be utilized to produce a dressing of the present disclosure include hydrophilic thermoplastic materials having a high moisture vapor transmission rate ("MVTR") from about 400 gr/m²/24-hours to about 3000 gr/m²/24-hours, in embodiments from about 500 gr/m²/24-hours to about 2000 gr/m²/24-hours. Such materials are within the purview of those skilled in the art and include, in embodiments, from about 5% to about 95% by weight water when hydrated, in embodiments from about 10% to about 50% by weight water when hydrated, in other embodiments from about 20% to about 40% by weight water when hydrated. Suitable hydrophilic materials include polymers such as cross-linked polyvinyl alcohol, cross-linked polyvinyl pyrrolidone, hydrophilic polyurethanes, hydrophilic hydroxyalkyl esters of poly(meth)acrylic acid and copolymers thereof, hydrophilic polyether-polyamide polymers, hydrophilic and water insoluble cellulosic derivatives such as cellulose acetate and cellulose acetate-propionate, as well as combinations of the foregoing.

Cross-linked polymers which may be utilized as the hydrophilic material may be cross-linked during the polymerization reaction or afterwards using a polyfunctional group such as a polyisocyanate.

In some embodiments, the hydrophilic layer may include a hydrophilic polymer such as cross-linked and/or linear hydrophilic polyurethanes. In embodiments, suitable hydrophilic materials include thermoplastic polyurethanes. Specific polyurethanes which may be utilized to form the hydrophilic layer include linear polyether polyurethanes formed from polyethylene glycol, polypropylene glycol, or combinations thereof with a diisocyanate and optionally an ethanediol or ethylene diamine as chain extender. Other hydrophilic polyurethanes which may be utilized include, for example, polyether thermoplastic polyurethanes including those derived from aromatic diisocyanates such as DUREFLEX^{®} RPT1700S (available from Deerfield Urethane).

This first hydrophilic layer of a dressing of the present disclosure may have a thickness from about 0.0102mm (0.4 mils) to about 0.038mm (1.5 mils), in embodiments from about 0.0152mm (0.6 mils) to about 0.0254mm (1 mils), with a thickness of about 0.0203mm (0.8 mils) being utilised in some embodiments. In embodiments, the first hydrophilic layer may include a monolithic, aromatic, thermoplastic polyurethane film. Such materials may be advantageously used in a dressing of the present disclosure adjacent a skin break or wound site, as monolithic films are excellent viral and bacterial barriers at thicknesses greater than or equal to about 0.0102mm (0.4 mils).

In embodiments the first layer may possess a coating. The coating may be found on any portion of the layer or, in some embodiments, may be on the side of the layer which will be placed adjacent a patient's skin. Suitable coatings are within the purview of those skilled in the art and include, for example, an adhesive such as a medical grade adhesive or a hydrogel. Suitable adhesives include any pressure sensitive adhesive which is medically accepted and skin friendly, including acrylic, hydrocolloid, hydrogel, polyurethane and silicone based adhesives, as well as combinations thereof.

Any coating may be applied to the hydrophilic layer utilizing means within the purview of those skilled in the art and may be continuous, semi-continuous, non-continuous, and the like. Thus, in embodiments, the coating may be an adhesive selected and applied so that the first hydrophilic layer has a desired level of adhesion to skin and MVTR.

In embodiments, it may be advantageous for the hydrophilic layer, and any coating applied thereto, including a hydrogel or adhesive, to be transparent.

The second layer of a dressing of the present disclosure includes a hydrophobic layer or film. Suitable hydrophobic layers may have a moisture vapour transmission rate ("MVTR") from about 250 gr/m²/24-hours to about 1000 gr/m²/24-hours, in embodiments from about 400 gr/m²/24-hours to about 750 gr/m²/24-hours. Suitable materials to use as the hydrophobic layer or film are within the purview of those skilled in the art and include, for example, breathable olefins, elastomeric co-polyesters, and urethanes. Specific examples of suitable hydrophobic materials include aromatic polyester urethanes such as DUREFLEX^{®} U04 (from Deerfield Urethane). This second hydrophobic layer may have a thickness from about 0.0102mm (0.4 mils) to about 0.127mm (5 mils), in embodiments from about 0.0254mm (1 mils) to about 0.102mm (4 mils).

In embodiments, the hydrophobic layer may be centered over the first hydrophilic layer and may be larger than the first layer so that the perimeter of the hydrophobic layer may form a uniform extended flange around and extending beyond the outer edge of the first hydrophilic layer. This construction may permit the use of an adhesive onto the extended flange portion of the hydrophobic layer to promote secure perimeter adherence upon application of a dressing of the present disclosure to skin. In other embodiments, the hydrophobic layer may be placed over the hydrophilic layer so that the hydrophobic layer is not centered, but rather off-set to one or more sides of the dressing.

In embodiments, the length of the extended flange, that is, the length by which the perimeter of the hydrophobic layer extends beyond the perimeter of the hydrophilic layer, may be from about 0.794mm (1/32 inch) to about 19.05mm (¾ inch), in embodiments from about 1.588mm (1/16 inch) to about 12.7mm (½ inch), with 6.35mm (¼ inch) being utilised in some embodiments. In embodiments, the extended flange of the hydrophobic layer may possess any adhesive described above on the side adjacent the patient's skin to enhance adherence of a dressing of the present disclosure to tissue.

In embodiments, the central portion of the hydrophobic layer may be removed, thereby forming a window in the dressing of the present disclosure. The removal of a central portion of the hydrophobic layer may result in the formation of an inside edge within the hydrophobic layer which should be from about 1.588mm (1/16 inch) to about 19.05mum (¾ inch) from the outer perimeter of the hydrophilic layer, in embodiments from about 3.175mm (1/8 inch) to about 12.7mm (½ inch) from the outer edge of the first hydrophilic layer.

The removal of a central portion of the hydrophobic layer may assist a dressing of the present disclosure in maintaining a desired level of flexibility, conformability, and MVTR. Moreover, such a window, when utilized with a transparent hydrophilic layer, would permit viewing of a wound site to which the dressing of the present disclosure has been applied.

Dressings of the present disclosure possesses additional optional layers. For example, in embodiments, a release layer is applied to the hydrophilic layer and any adhesive coating thereon to protect the adhesive layer. In embodiments, such a release layer may also cover the portion of the hydrophobic layer extending beyond the edge of the hydrophilic layer; such a covering may be especially useful where the portion of the hydrophobic layer extending beyond the edge of the hydrophilic layer possesses an adhesive thereon. Any release layer commercially available and/or within the purview of those skilled in the art may be utilized.

A third layer is applied to the hydrophobic layer of a dressing of the present disclosure. Such a layer may, in embodiments, be non-breathable and hydrophobic. Such a layer, in embodiments referred to as a delivery layer, is utilized to provide a means for single-handed, wrinkle free application of a dressing of the present disclosure. Suitable materials which may be utilized to form this delivery layer include thermal plastic films including, but not limited to, olefins, polyesters, copolymers thereof and combinations thereof. In embodiments, a polyethylene/ethylene vinyl acetate (EVA) blend may be utilized as the delivery layer. Other materials which may be utilized to form this delivery layer include monolayer metallocene films, which may have a smooth or matte surface finish, and polyethylene/vinyl acetate copolymer-coated super calendered bleached kraft (commercially available as 1-80BKG-157 from Loparex, Inc., or similar materials).

In embodiments, the hydrophobic layer may be devoid of fillers and other processing aids sometimes utilized in conventional dressings. Such fillers and processing aids may inhibit the ability of the hydrophobic layer to bond to the delivery layer.

Methods for attaching the hydrophilic layer to the hydrophobic layer and, similarly, attaching the delivery layer to the hydrophobic layer are within the purview of those skilled in the art and include, for example, thermal bonding processes. In embodiments the thermal bonding process may include heating the hydrophilic/hydrophobic combination of layers to a temperature from about 115.6°C (240° F) to about 172.8°C (343° F), in embodiments from about 118.3°C (245° F) to about 129.4°C (265° F), for a period of time from about 0.125 seconds to about 0.025 seconds, in embodiments from about 0.05 seconds to about 0.035 seconds. This heating process may result in the thermal bonding of the hydrophilic layer to the hydrophobic layer. Similarly, bonding of the delivery layer may be accomplished by heating the hydrophobic layer or hydrophilic/hydrophobic combination of layers and the delivery layer to a temperature from about 107.2°C (225° F) to about 148.9°C (300° F), in embodiments from about 126.7 °C (260° F) to 137.8 °C (280° F), for a period of time from about 0.45 seconds to about 0.0025 seconds, in embodiments from about 0.225 seconds to about 0.003 seconds.

The heating of the various layers utilized to form a dressing of the present disclosure may use a plurality of heated rolls, with at least two arranged to provide compressive forces to attain bonding. In embodiments, it may be desirable that at least one of the rolls be covered with a soft thermally stable surface, in embodiments a silicone rubber, though other materials within the purview of those skilled in the art may be utilized. It is envisioned that the soft surface could have a durometer (surface hardness), from about 40 shore A to about 100 shore A, in embodiments from about 60 shore A to about 80 shore A, with a durometer of about 70 shore A being utilized in some embodiments.

The delivery layer may, in embodiments, possess a size and shape comparable to that of the hydrophobic layer and, in embodiments, may cover the entire area of both the hydrophilic layer and the hydrophobic layer. In embodiments, a central portion of the delivery layer may also be removed. The central portion of the delivery layer which is removed may be of any shape; in embodiments, the dimensions of the central portion removed from the delivery layer are similar to the dimensions of the portion removed from the hydrophobic layer, i.e., the window described above. In some embodiments the shape and dimension of the central portion removed from the delivery layer may be identical to the shape and dimension of the central portion removed from the hydrophobic layer so that the window formed in the delivery layer is contiguous with the window formed in the hydrophobic layer.

In embodiments an additional fourth layer may be utilized in a dressing of the present disclosure. Such a layer may, in embodiments, be constructed of a nonwoven material, in embodiments a cellulosic material. This fourth layer may be coated on one of its sides with a suitable adhesive, including those adhesives described above for use on the hydrophilic layer and/or the hydrophobic layer and may, in embodiments, be referred to as an adhesive tape. This fourth layer may be positioned between the delivery layer and the hydrophobic layer so that the side possessing the adhesive is adjacent the delivery layer and the side lacking adhesive is in contact with the hydrophobic layer. This layer may, in embodiments, be positioned along one edge of the delivery layer, such that its outer edge is coextensive with the outer edge of the delivery layer (as noted above, the outer edge of the delivery layer may extend beyond the outer edge of the hydrophobic layer). The inner edge of the fourth layer may, in embodiments, overlap the outer edge of the hydrophobic layer by at least about 1.588mm (1/16 of an inch). The amount of overlap may vary, in embodiments from about 1.588mm (1/16 inch) to about 12.7mm (½ inch), in embodiments from about 3.175mm (1/8 inch) to about 6.35mm (1/4 inch).

Where present, the fourth layer may serve several purposes. In embodiments, the fourth layer may facilitate the removal of the delivery layer from the hydrophobic layer upon application of a dressing of the present disclosure, by providing an initial disruption of the surface bond between the delivery layer and the hydrophobic layer and providing a means to grasp thereby facilitating separation of the delivery layer from the hydrophobic layer via peeling. Moreover, in embodiments, the fourth layer may be separated from the delivery layer via peeling at which time it may function as a change documentation tool, i.e., its presence may be utilized as evidence of a change of dressings or, in embodiments, notations including the date and time of application of the dressing may be placed on the fourth layer after removal, which may be retained as evidence of the change of the dressing. Moreover, the fourth layer could be further adapted to better facilitate the peel of said layer by applying any adhesive to the fourth layer in a manner to provide a non-coated edge or edges. In other embodiments, the inboard edge of the fourth layer may be folded over onto the adhesive side of the fourth layer. This would produce a non-adhered flap to facilitate removal. In yet other embodiments, the fourth layer may be subjected to a controlled depth die cutting process that would cut any protective release liner attached to the adhesive on the fourth layer prior to its attachment to the third layer. The cut would allow a portion of the release liner to remain while the balance is removed. The remaining portion would provide a non-adhered flap that would facilitate tape peel.

Dressings of the present disclosure may possess varying geometries and configurations. In addition, in embodiments, notches, slits, clearance holes, and similar perimeter modifications may be made to a dressing of the present disclosure at one or more locations of the perimeter of a dressing to facilitate the use of a dressing of the present disclosure with various other medical devices, including ported and non-ported intravenous needles and similar devices which may be utilized in short peripheral intravenous applications. Where present, it may be advantageous for the various layers of a dressing of the present disclosure to possess a notch, etc. in the same location so that the notches of the various layers are contiguous and result in a dressing possessing a notch in one location thereon.

In yet other embodiments, an additional layer may be applied to a dressing of the present disclosure between the hydrophilic layer and the hydrophobic layer. Such a layer may be utilized to further stabilize a dressing of the present disclosure and provide additional structure thereto and may be referred to, in embodiments, as a stabilizer layer. Such stabilization may be desirable for various procedures, including the use of a dressing of the present disclosure in conjunction with central venous catheters (CVC), dialysis, and/or pulmonary artery (PA) catheters. Where utilized, this additional stabilizer layer may be placed between the hydrophilic layer and the hydrophobic layer, in embodiments at the periphery of the hydrophobic layer and hydrophilic layer where modifications such as notches, slits, and the like may be present. A stabilizer layer may be constructed of appropriate materials within the purview of those skilled in the art capable of providing the desired structure and rigidity to a dressing of the present disclosure. Both woven and non-woven materials are contemplated, including gauze, cloth, thermal bonded polypropylene, spunbonded polyproplene, nylon (CEREX™), hydroentangled polyester (SONTARA™), nonwoven cellulosic acetate, woven taffeta acetate, and combinations thereof.

Where present, the stabilizer layer may possess thereon any adhesive coating described above as suitable for any other layer of a dressing of the present disclosure, to facilitate its application and adherence to the hydrophobic layer and/or the hydrophilic layer. The strength of any adhesive applied to the stabilizer layer should be, at a minimum, equivalent to the strength of any adhesive utilized to adhere the hydrophobic layer to the hydrophilic layer. The stabilizer layer may, in embodiments, provide a rigid construction to assist in keeping any catheter and/or intravenous line from moving excessively. This may be beneficial as excessive movement of a catheter or intravenous line may cause both patient discomfort and other complications.

A stabilizer layer may possess a configuration similar to the hydrophobic layer, i.e., with a window removed from a central portion thereof. In other embodiments, a stabilizer layer may possess a strip configuration and may be located at one side of the hydrophilic layer and hydrophobic layer at the perimeter of these two layers.

In yet other embodiments, a dressing of the present disclosure may include an absorbent material. The absorbent material may be included as a component of any other layer of the dressing of the present disclosure or may be included as a separate layer of a dressing of the present disclosure. In some embodiments, it may be advantageous for the hydrophilic layer of the present disclosure to possess an absorbent material. Such an absorbent material may be applied to any locus of the hydrophilic layer, but typically is present in the surface plane of the hydrophilic layer. As described above, in embodiments the absorbent material may be applied to the hydrophilic layer as a separate absorbent layer. In other embodiments, the absorbent material may be located at the perimeter of the hydrophilic layer, for example, by being applied only to the perimeter of the hydrophilic layer, or where utilized as a separate layer, by having a central portion removed therefrom thereby forming a window in the absorbent layer similar to the window which may be present in the other layers as described above.

The absorbent material may help control minor leakage at the wound site, or at the site of insertion of a needle, depending upon the circumstances of the application of a dressing of the present disclosure. The ability to absorb such fluids may extend the life of the dressing by minimizing the deterioration of the dressing or any component thereof, including adhesives, which may otherwise occur in the presence of body fluids including any wound exudate. Suitable materials which may be utilized as the absorbent material include those currently utilized with dressings and/or the treatment of wounds including, for example, cotton, alginate, rayon, cellulose, urethanes, hydrogels, hydrocolloids, polyethylene oxides, and superabsorbent polymers such as sodium and aluminum salts of starch grafted copolymers of acrylates and acrylamides, combinations thereof, as well as polyacrylate salts. In some embodiments, superabsorbent polymers which may be utilized include hydrophilic cellulose derivatives that have been partially cross-linked to form a three dimensional structure. Suitable cross-linked cellulose derivatives include those of the hydroxy lower alkyl celluloses, wherein the alkyl group contains from about 1 to about 6 carbon atoms, for example, hydroxyethyl cellulose or hydroxypropylcellulose, or the carboxy-celluloses such as, for example, carboxymethyl hydroxyethyl cellulose or carboxy methylcellulose. Salts of such polymers, for example a partially cross-linked sodium carboxy methylcellulose polymer, may be utilized in embodiments.

Other absorbent materials which can be used include methylcellulose, guar gum, pectin, karaya gum, chitosan, agar, acacia powder, carrageenan, gelatin and combinations thereof. The absorbent material may be in any suitable form including, but not limited to, woven or nonwoven webs, fibers, powders, pastes, foams, gels, or any other form that may be incorporated in another layer or applied as a separate absorbent layer in forming a dressing of the present disclosure. In embodiments, the absorbent material may possess natural antimicrobial properties.

Dressings of the present disclosure may possess any configuration, which may be adjusted depending upon the desired use of the dressing and the part of the body to which the dressing is to be applied.

In other embodiments, a tape may be utilized to further assist in attaching a dressing of the present disclosure to a patient. Such tapes are within the purview of those skilled in the art and are commercially available. Any medically acceptable tape utilized to adhere dressings to a patient may be utilized.

Dressings of the present disclosure have a thinner profile than conventional dressings. A dressing of the present disclosure may have a thickness from about 0.076mm (3 mils) to about 0.279mm (11 mils), in embodiments from about 0.117mm (4.6 mils) to about 0.178mm (7 mils).

Turning now to the figures, various configurations of dressings of the present disclosure are described. As set forth in Figure 1, a dressing of the present disclosure includes a hydrophilic film 10 which may possess a high MVTR from about 400 gr/m²/24-hour to about 3000 gr/m²/24-hour, in embodiments from about 500 gr/m²/24-hour to about 2000 gr/m²/24-hour. Hydrophilic film layer 10 may, in embodiments, possess an adhesive coating thereon (not shown) as described above. Hydrophobic layer 20 may be placed adjacent to hydrophilic layer 10. In embodiments, central portion of hydrophobic layer 20 may be removed, thereby forming a window 22 in hydrophobic layer 20. Hydrophobic layer 20 may possess a low to moderate MVTR of from about 250 gr/m²/24-hour to about 1000 gr/m²/24-hour, in embodiments from about 400 gr/m²/24-hour to about 750 gr/m²/24-hour. Delivery layer 30 may be adjacent hydrophobic layer 20 on the side of hydrophobic layer 20 opposite hydrophilic layer 10. In embodiments delivery layer 30 may be thermally bonded to hydrophobic layer 20 and hydrophilic layer 10 (through the window 22 of hydrophobic layer 20). Delivery layer 30 may also possess printing or markings thereon. Delivery layer 30 may possess edges 31 and 33 which may be manually gripped to facilitate removal of delivery layer 30 from hydrophobic layer 20. In some embodiments, an adhesive covered tape 40 may be attached to delivery layer 30 so that it overlaps the edge of the hydrophobic layer 20. Adhesive tape 40 may be utilized to facilitate the removal of delivery layer 30 from hydrophobic layer 20. Finally, a release layer 50 may be placed adjacent to hydrophilic layer 10 on the side opposite of hydrophobic layer 20 wherein the release layer 50 is attached to hydrophilic layer 10 and hydrophobic layer 20 so that it covers hydrophilic layer 10, hydrophobic layer 20, any adhesive on either such layer, delivery layer 30, and adhesive tape 40 prior to application of the dressing of the present disclosure to a patient. Upon removal of release layer 50, the dressing of the present disclosure may be applied to a patient so that hydrophilic layer 10 and hydrophobic layer 20 may remain affixed to a patient.

Figure 2 depicts an alternate dressing of the present disclosure having the same layers as described above in Figure 1, except that the delivery layer 30 of the dressing of the present disclosure may have a central portion removed forming a window 32 in delivery layer 30. In embodiments, the window 32 of delivery layer 30 is of the same dimensions and thus is contiguous with the window 22 of hydrophobic layer 20. Due to the presence of window 32, in this embodiment delivery layer 30 is thermally bonded to hydrophobic layer 20, but not hydrophilic layer 10. Prior to application to a patient, release layer 50 is in contact with hydrophilic layer 10 and hydrophobic layer 20.

Figure 3 depicts another dressing of the present disclosure, having the same basic configuration as the dressing of Figure 1, but with notches in the various layers so that a dressing of the present disclosure may be utilized to help secure a needle during a CVC procedure, dialysis, a PA procedure, that is, a catheterization of a pulmonary artery, and the like. In this embodiment, hydrophilic layer 10 possesses a notch 14; hydrophobic layer 20 possesses window 22a and notch 24; delivery layer 30 possesses a notch 34, and adhesive tape 40 may either possess a notch (not shown) or be in two separate pieces, 42 and 44. As depicted in Figure 3, the layers are positioned so that the notches are contiguous with each other thereby forming a dressing with a single notch therein (that is, notch 34, is placed over adhesive pieces 42 and 44 so that the notch or gap between pieces 42 and 44 is contiguous with notch 34, and the notch or gap between pieces 42 and 44 is over notch 24 which, in turn, is over notch 14). As depicted in Figure 3, notch 24 in hydrophobic layer 20 may alter the configuration of window 22a therein so that the notch 24 and window 22a are not connected and a portion of hydrophobic layer 20 remains between notch 24 and window 22a. Prior to application to a patient, release layer 50 is in contact with hydrophilic layer 10 and hydrophobic layer 20.

Another dressing of the present disclosure is set forth in Figure 4. This is the same basic configuration as the dressing of Figure 3, but delivery layer 30 also possesses a window 32a therein. As depicted in Figure 4, notch 24 in hydrophobic layer 20 may alter the configuration of window 22a therein so that the notch 24 and window 22a are not connected and a portion of hydrophobic layer 20 remains between notch 24 and window 22a. Similarly, notch 34 in delivery layer 30 may alter the configuration of window 32a therein so that the notch 34 and window 32a are not connected and a portion of delivery layer 30 remains between notch 34 and window 32a. Window 32a in delivery layer 30 and window 22a in hydrophobic layer may be contiguous as depicted in Figure 4.

Figure 5 depicts another dressing of the present disclosure similar to the dressing of Figure 1, with the addition of a stabilizer layer. The dressing of Figure 5 includes hydrophilic layer 10, hydrophobic layer 20 possessing window 22, delivery layer 30, adhesive tape 40, and release layer 50. The dressing of Figure 5 further possesses stabilizer layer 60 having a window 62 located between hydrophilic layer 10 and hydrophobic layer 20. Stabilizer layer 60 may be made of a breathable material and possess an adhesive coating which attaches stabilizer layer 60 to the adhesive side of hydrophobic layer 20 and the non-adhesive side of hydrophilic layer 10. In this embodiment, release layer 50 is attached to hydrophilic layer 10 and stabilizer layer 60.

Figure 6 depicts an alternate embodiment of the dressing of Figure 5, wherein the dressing possesses a window 32 in delivery layer 30. Thus the dressing of Figure 6 possesses hydrophilic layer 10, hydrophobic layer 20 possessing window 22, delivery layer 30 possessing window 32, adhesive tape 40, release layer 50, and stabilizer layer 60 having a window 62 located between hydrophilic layer 10 and hydrophobic layer 20. In this embodiment, release layer 50 is attached to hydrophilic layer 10 and stabilizer layer 60 prior to removal of release layer 50 and the application of the dressing to a patient.

Figure 7 depicts an alternate embodiment of the dressing of Figure 5. In this embodiment, the stabilizer layer is not co-extensive with the hydrophobic layer 20 but, instead, is present as a stabilizer strip 66 found on one side of a dressing of the present disclosure. The dressing of Figure 6 thus includes hydrophilic layer 10, hydrophobic layer 20 possessing window 22, delivery layer 30, adhesive tape 40, and release layer 50. The dressing of Figure 6 possesses stabilizer layer in the form of a strip 66 located between hydrophilic layer 10 and hydrophobic layer 20 on one side of the dressing at the perimeter of the hydrophilic layer 10 and hydrophobic layer 20. Stabilizer strip 66 may be made of a breathable material and possess an adhesive coating which attaches stabilizer strip 66 to the adhesive side of hydrophobic layer 20 and the non-adhesive side of hydrophilic layer 10. In this embodiment, release layer 50 is attached to hydrophilic layer 10, hydrophobic layer 20 and stabilizer strip 66 prior to application of the dressing to a patient.

Figure 8 depicts an alternate embodiment of the dressing of Figure 7, wherein the dressing possesses a window 32 in delivery layer 30. Thus the dressing of Figure 7 possesses hydrophilic layer 10, hydrophobic layer 20 possessing window 22, delivery layer 30 possessing window 32, adhesive tape 40, release layer 50, and stabilizer strip 66 located between hydrophilic layer 10 and hydrophobic layer 20 on one side of the dressing. In this embodiment, release layer 50 is attached to hydrophilic layer 10, hydrophobic layer 20 and stabilizer strip 66 prior to application of the dressing to a patient.

The dressing of Figure 9 combines the dressing of Figure 3 with the stabilizer strip 66 of Figure 7. In this embodiment, hydrophilic layer 10 possesses a notch 14; hydrophobic layer 20 possesses window 22a and notch 24; delivery layer 30 possesses a notch 34, and adhesive tape 40 may either possess a notch (not shown) or be in two separate pieces, 42 and 44. As depicted in Figure 9, notch 24 in hydrophobic layer 20 may alter the configuration of window 22a therein so that the notch 24 and window 22a are not connected and a portion of hydrophobic layer 20 remains between notch 24 and window 22a. The dressing of Figure 9 further includes stabilizer strip 66a possessing notch 64 therein. As with the dressings with notches described above, the notch 64 of stabilizer strip 66a is placed so that it is contiguous with the notches found in the other layers of the dressing of the present disclosure (i.e., notches 14, 24, 34, and the notch in adhesive tape 40 or the gap formed between adhesive tape segments 42 and 44). In embodiments, stabilizer strip 66a may be of a sigmoidal configuration so that stabilizer strip 66a has indents 61 and 63 in the side of strip 66a opposite the side possessing notch 64. Prior to application to a patient, release layer 50 is in contact with hydrophilic layer 10, hydrophobic layer 20, and stabilizer strip 66a.

The dressing of Figure 10 combines the dressing of Figure 4 with the stabilizer strip of Figure 7. In this embodiment, hydrophilic layer 10 possesses a notch 14; hydrophobic layer 20 possesses window 22a and notch 24; delivery layer 30 possesses window 32a and notch 34, and adhesive tape 40 may either possess a notch (not shown) or be in two separate pieces, 42 and 44. As depicted in Figure 10, notch 24 in hydrophobic layer 20 may alter the configuration of window 22a therein so that the notch 24 and window 22a are not connected and a portion of hydrophobic layer 20 remains between notch 24 and window 22a. Similarly, notch 34 in delivery layer 30 may alter the configuration of window 32a therein so that the notch 34 and window 32a are not connected and a portion of delivery layer 30 remains between notch 34 and window 32a. The dressing of Figure 10 further includes stabilizer strip 66a possessing notch 64 therein. In embodiments, stabilizer strip 66a may be of a sigmoidal configuration so that stabilizer strip 66a has indents 61 and 63 in the side of strip 66a opposite the side possessing notch 64. Prior to application to a patient, release layer 50 is in contact with hydrophilic layer 10, hydrophobic layer 20, and stabilizer strip 66a.

Figure 11 depicts an alternate dressing of the present disclosure. The dressing of Figure 11 is the dressing of Figure 1 with an absorbent layer placed between the hydrophilic layer and the skin. Thus, the dressing of Figure 11 includes hydrophilic layer 10, hydrophobic layer 20 possessing window 22 therein, delivery layer 30, adhesive tape 40, release layer 50, and absorbent layer 70. As depicted in Figure 11, absorbent layer 70 may have a central portion removed therefrom thereby forming window 72 in absorbent layer 70. The absorbent layer 70 is attached to the side of the hydrophilic layer 10 possessing an adhesive and is placed between hydrophilic layer 10 and release layer 50 so that absorbent layer 70 comes into contact with a patient's skin upon removal of the release layer and application of a dressing of the present disclosure to the patient. Prior to application to a patient, release layer 50 is in contact with hydrophilic layer 10, hydrophobic layer 20, and absorbent layer 70.

Figure 12 is another embodiment of the dressing of Figure 11, wherein the delivery layer possesses a window as described in Figure 2. Thus, the dressing of Figure 12 possesses hydrophilic layer 10, hydrophobic layer 20 possessing window 22 therein, delivery layer 30 possessing window 32 therein, adhesive tape 40, release layer 50, and absorbent layer 70 possessing window 72 therein. Prior to application to a patient, release layer 50 is in contact with hydrophilic layer 10, hydrophobic layer 20, and absorbent layer 70.

Figure 13 provides yet another embodiment of a dressing of the present disclosure. The dressing of Figure 13 includes hydrophilic layer 10 possessing a notch 14, hydrophobic layer 20a possessing a notch 24, delivery layer 30 possessing notch 34, and release layer 50. Rather than a window therein, hydrophobic layer 20a is configured as depicted in Figure 13 so that it is adjacent to a majority of the perimeter of hydrophilic layer 10 and includes the notch 24 overlapping notch 14, but does not possess a window or an otherwise solid configuration.

The present disclosure also relates to the use of dressings according to the present disclosure in medicine, for example, as wound dressings, bandages or supports.

Dressings of the present disclosure may contain, if desired, one or more medicinal agents. In embodiments, such medicinal agents may elute from the dressing of the present disclosure at the site of application. As used herein, "medicinal agent" is used in its broadest sense and includes any substance or mixture of substances that have clinical use. Consequently, medicinal agents may or may not have pharmacological activity per se, e.g., a dye. Examples of classes of medicinal agents which may be combined or mixed into the foam of the present disclosure include antimicrobials, analgesics, antipyretics, anesthetics, antiepileptics, antihistamines, anti-inflammatories, diagnostic agents, sympathomimetics, cholinomimetics, antimuscarinics, antispasmodics, hormones, growth factors, muscle relaxants, antineoplastics, immunosuppressants, steroids, polysaccharides, and enzymes. It is also intended that combinations of medicinal agents may be used.

Suitable antimicrobial agents which may be included as a medicinal agent in the dressings of the present disclosure include triclosan, also known as 2,4,4'-trichloro-2'-hydroxydiphenyl ether, biguanides including polyhexamethylne biguanide and chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a medicinal agent in the dressings of the present disclosure.

Other medicinal agents which may be included as a medicinal agent in the dressings of the present disclosure include: local anesthetics; parasympathomimetic agents; tranquilizers; sulfonamides; vitamins; antimalarials; anti-migraine agents; anti-parkinson agents such as L-dopa; anti-spasmodics; anticholinergic agents (e.g. oxybutynin); cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; estrogens; antibacterials; antifungals; antivirals; anticoagulants; anticonvulsants; antidepressants; immunological agents; hormones and hormone analogs (e.g., growth hormone, adrenocorticotropic hormone and luteinizing hormone releasing hormone (LHRH)); vaccines (e.g., tumoral, bacterial and viral antigens); somatostatin; antigens; blood coagulation factors; growth factors (e.g., nerve growth factor, insulin-like growth factor); protein inhibitors, protein antagonists, and protein agonists; nucleic acids, such as antisense molecules, DNA and RNA; oligonucleotides; and ribozymes. As noted above, in embodiments combinations of medicinal agents may be utilized.

The amount of medicinal agent present will depend upon the particular medicinal agent chosen, but may be in an amount from about 10 parts per million (ppm) to about 10,000 ppm.

While medicinal agents may be incorporated in or applied to any layer utilized to form a dressing of the present disclosure, in embodiments the medicinal agents may be applied to those layers and/or components which come into contact with a patient's skin. Such layers include, for example, the hydrophilic layer, adhesive layers applied thereto, and/or to the periphery of the hydrophobic layer, and any absorbent material included in the surface plane or at the periphery of the hydrophilic layer, or applied to the hydrophilic layer as a separate absorbent layer.

Medicinal agent(s) or other additives may be incorporated into a dressing of the present disclosure by any method within the purview of those skilled in the art. In embodiments, the agent(s) or other additives may be incorporated into the dressing by addition of agent(s) or other additives into the materials utilized in forming the layers of the dressing before reacting and forming the material utilized to form the specific layer. In other embodiments, the agent(s) or other additives may be incorporated into the dressing by separately introducing the agent(s) or additives as the various layers of a dressing of the present disclosure are combined. In yet other embodiments, the agent(s) or other additives may be incorporated into the dressing by a padding process after the dressing is formed, for example by applying the agent(s) or additives to the dressing or any layer thereof by saturating the dressing or layer in a trough or similar vessel and then squeezing the saturated dressing or layer through pressure rollers to achieve a uniform application of the agent(s) or additives and incorporation of the agent(s) and/or additives both upon the surface of the dressing and/or layer or within the dressing or layer itself.

In yet other embodiments, agent(s) or other additives may be applied as a coating to the dressings of the present disclosure, either by separate application of said agent(s) or other additives in a solvent and then evaporating the solvent or by their inclusion in an additional layer utilized to form a dressing of the present disclosure. Such layers include any additional layers such as backing layers, including polyurethane backing layers, or any additional nonwoven layer, fibrous layer, or adhesive utilized in combination with a dressing of the present disclosure. In embodiments, agent(s) or additives may be included in a separate coating applied to a dressing of the present disclosure. Such coatings may be made of any biocompatible material, including both natural and synthetic polymers, copolymers, hydrogels, and the like. Such coatings may also be applied to any backing layer, adhesive layer, or any other layer of a dressing of the present disclosure.

In embodiments, coating materials may include peptides or proteins including, but not limited to, albumin, collagen, fibrin, elastin and the like. Other coating materials which may be utilized include polysaccharides such as chitosan, alginate, hyaluronic acid and the like. In other embodiments, synthetic polymers may be utilized as the coating material. Such polymers include, for example, polyesters, polyethers, polycarbonates, and polyanhydrides. Suitable polyesters which may be utilized are within the purview of those skilled in the art and include, for example, trimethylene carbonate, ∈-caprolactone, p-dioxanone, glycolide, lactide, 1,5-dioxepan-2-one, polybutylene adipate, polyethylene adipate, polyethylene terephthalate, and homopolymers and copolymers thereof. Suitable polyethers which may be utilized are within the purview of those skilled in the art and include, for example, polyethylene glycol, polypropylene glycol, polybutylene glycol, polytetramethylene glycol, polyhexamethylene glycol, homopolymers thereof and copolymers thereof. Suitable polycarbonates include, for example, tetramethylene carbonates, trimethylene carbonates, pentamethylene carbonates, homopolymers thereof, copolymers thereof, and the like.

Dressings of the present disclosure possess several advantages compared with conventional dressings. They may be applied with only one hand, and undergo minimal wrinkling, which is of great advantage to any medical personnel utilizing such dressings. They provide maximum MVTR to the wound/insertion area. They minimize the need for the use of additional skin adhesives and may be constructed so that the perimeter has greater adhesion, thereby reducing or preventing lift of the dressing. The lower degree of adhesion at the wound/insertion area minimizes wound trauma or catheter removal at dressing change, and any adhesive used thereon could be replaced with a hydrogel which, in embodiments, could possess a medicinal agent such as an antimicrobial. In other embodiments, if the hydrophilic layer is constructed of a transparent material, the wound/insertion site may be easily inspected without the need for removing the dressing of the present disclosure.

It will be understood that various modifications may be made to the embodiments disclosed herein. Therefore, the above description should not be construed as limiting, but merely as exemplifications of useful embodiments. Those skilled in the art will envision other modifications within the scope of the claims appended hereto.

## Claims

1. An article comprising:
at least one hydrophilic layer (10) having a skin-facing side and a side opposite thereto;
at least one hydrophobic layer (20) adjacent to the hydrophilic layer on the side opposite to the skin-facing side;
a delivery layer (30) adjacent to the hydrophobic layer on the side opposite to the hydrophilic layer; and
a release layer (50) on the side of the hydrophilic layer opposite to the hydrophobic layer,
wherein the hydrophobic layer possesses a window (22) therein formed by the removal of a central portion of the hydrophobic layer.

2. An article as claimed in claim 1, wherein the hydrophilic layer (10) is formed from a polymer selected from the group consisting of cross-linked polyvinyl alcohol, cross-linked polyvinyl pyrrolidone, hydrophilic polyurethanes, hydrophilic hydroxyalkyl esters of poly(meth)acrylic acid and copolymers thereof, hydrophilic polyether-polyamide polymers, hydrophilic and water insoluble cellulosic derivatives, and combinations thereof.

3. An article as claimed in claim 1, wherein the hydrophilic layer (10) comprises a hydrophilic polyurethane formed from polyethylene glycol, polypropylene
glycol, or combinations thereof with a diisocyanate and optionally an ethanediol or ethylene diamine.

4. An articleas claimed in claim 1, wherein the hydrophilic layer (10) has a moisture vapour transition rate from substantially 400 gr/m²/24-hour to substantially 3000 gr/m²/24-hour.

5. An article as claimed in claim 4, wherein the hydrophilic layer (10) has a thickness from substantially 0.0102mm (0.4 mils) to substantially 0.038mm (1.5 mils).

6. An article as claimed in claim 1, wherein the hydrophobic layer (20) is selected from the group consisting of breathable olefins, elastomeric co-polyesters, urethanes, and combinations thereof.

7. An article as claimed in claim 1, wherein the hydrophobic layer (20) comprises a polyester urethane.

8. An article as claimed in claim 1, wherein the hydrophobic layer (20) has a moisture vapour transition rate from substantially 250 gr/m²/24-hour to substantially 1000 gr/m²/24-hour.

9. An article as claimed in claim 8, wherein the hydrophobic layer (20) has a thickness from substantially 0.0102mm (0.4 mils) to substantially 0.127mm (5 mils).

10. An article as claimed in claim 1, wherein the skin-facing side of the hydrophilic layer (10) and the hydrophobic layer (20), or both, has a coating on at least a portion thereof comprising a medically accepted adhesive selected from the group consisting of acrylics, hydrocolloids, hydrogels, polyurethanes, silicones, and combinations thereof.

11. An article as claimed in claim 1, wherein the perimeter of the hydrophobic layer (20) extends beyond the perimeter of the hydrophilic layer (10) by a distance from substantially 0.794mm (1/32 inch) to substantially 19.05mm (¾ inch).

12. An article as claimed in claim 1, wherein the delivery layer (30) comprises olefins, polyesters, copolymers thereof, and combinations thereof.

13. An article as claimed in claim 1, wherein the delivery layer (30) comprises a polyethylene/ethylene vinyl acetate blend.

14. An article as claimed in claim 1, wherein the delivery layer (30) possesses a window (32) therein formed by the removal of a central portion of the delivery layer.

15. An article as claimed in claim 1, further comprising an adhesive tape (40) between the hydrophobic layer (20) and the delivery layer (30).

16. An article as claimed in claim 1, further comprising a stabiliser layer (60) between the hydrophilic layer (10) and the hydrophobic layer (20).

17. An article as claimed in claim 16, wherein the stabiliser layer (60) is formed of a material selected from the group consisting of gauze, cloth, thermal bonded polypropylene, spunbonded polypropylene, nylon, hydroentangled polyester, nonwoven cellulosic acetate, woven taffeta acetate.

18. An article as claimed in claim 1, further comprising an absorbent material (70) on the skin-facing side of the hydrophilic layer (10).

19. An article as claimed in claim 18, wherein the absorbent material (70) is selected from the group consisting of cotton, alginate, rayon, cellulose, urethanes, hydrogels, hydrocolloids, polyethylene oxides, superabsorbent polymers, and combinations thereof.

20. An article as claimed in claim 1, wherein the hydrophilic layer (10), the hydrophobic layer (20), and the delivery layer (30) possess notches (14, 24, 34) therein which are contiguous with each other.

21. An article as claimed in claim 1, wherein the article has a thickness from substantially 0.076mm (3 mils) to substantially 0.279mm (11 mils).

22. An article as claimed in claim 1, further comprising a medicinal agent selected from the group consisting of antimicrobials, analgesics, antipyretics, anesthetics, antiepileptics, antihistamines, anti-inflammatories, diagnostic agents, sympathomimetics, parasympathomimetics, cholinomimetics, antimuscarinics, antispasmodics, hormones, hormone analogs, growth factors, muscle relaxants, antineoplastics, immunosuppressants, steroids, polysaccharides, enzymes, tranquilizers, sulfonamides, vaccines, vitamins, antimalarials, anti-migraine agents, anti-parkinson agents, anticholinergics, cardiovascular agents, alkaloids, narcotics, opioid receptor antagonists, anti-cancer agents, anti-convulsants, anti-emetics, antihistamines, prostaglandins, cytotoxic drugs, estrogens, antibacterials, antifungals, antivirals, anticoagulants, anticonvulsants, antidepressants, immunological agents, antigens, blood coagulation factors, protein inhibitors, protein antagonists, protein agonists, nucleic acids, oligonucleotides, ribozymes, and combinations thereof.

23. An article as claimed in claim 1, wherein
the hydrophilic layer (10) possesses a moisture vapour transition rate from substantially 400 gr/m²/24-hour to substantially 3000 gr/m²/24-hour and has a skin-facing side, and a side opposite thereto;
the hydrophobic layer (20) adjacent to the hydrophilic layer on the side opposite the skin-facing side possesses a moisture vapour transition rate from substantially 250 gr/m²/24-hour to substantially 1000 gr/m²/24-hour and has a window (22) therein formed by the removal of a central portion of the hydrophobic layer;
the delivery layer (30) adjacent to the hydrophobic layer on the side opposite the hydrophilic layer has a window (32) therein formed by the removal of a central portion of the delivery layer; and
wherein the windows in the hydrophobic layer and the delivery layer are contiguous with each other, and the article has a thickness from substantially 0.0.76mm (3 mils) to substantially 0.279mm (11 mils).

24. An article as claimed in claim 23, wherein the release layer (50) is on the side of the hydrophilic layer (10) opposite to the hydrophobic layer (20).

## Patentansprüche

1. Gegenstand, umfassend:
wenigstens eine hydrophile Schicht (10) mit einer hautseitigen Seite und einer dazu gegenüberliegenden Seite;
wenigstens eine hydrophobe Schicht (20), die der hydrophilen Schicht an der Seite, die der hautseitigen Seite gegenüberliegt, benachbart ist;
eine Abgabeschicht (30), die der hydrophoben Schicht an der Seite, die der hydrophilen Schicht gegenüberliegt, benachbart ist; und
eine Abziehschicht (50) an der Seite der hydrophilen Schicht, die der hydrophoben Schicht gegenüberliegt,
wobei die hydrophobe Schicht ein Fenster (22) aufweist, das durch Entfernen eines mittleren Teils der hydrophoben Schicht darin gebildet ist.

2. Gegenstand gemäß Anspruch 1, wobei die hydrophile Schicht (10) aus einem Polymer gebildet ist, ausgewählt aus der Gruppe bestehend aus vernetztem Polyvinylalkohol, vernetztem Polyvinylpyrrolidon, hydrophilen Polyurethanen, hydrophilen Hydroxyalkylestern von Poly(meth)acrylsäure und Copolymeren davon, hydrophilen Polyetherpolyamidpolymeren, hydrophilen und wasserunlöslichen Cellulosederivaten, und Kombinationen davon.

3. Gegenstand gemäß Anspruch 1, wobei die hydrophile Schicht (10) ein hydrophiles Polyurethan umfasst, gebildet aus Polyethylenglycol, Polypropylenglycol und Kombinationen davon mit einem Diisocyanat und gegebenenfalls einem Ethandiol oder Ethylendiamin.

4. Gegenstand gemäß Anspruch 1, wobei die hydrophile Schicht (10) eine Wasserdampf-Übergangsrate von im Wesentlichen 400 g/m²/24-Stunden bis im Wesentlichen 3000 g/m²/24-Stunden aufweist.

5. Gegenstand gemäß Anspruch 4, wobei die hydrophile Schicht (10) eine Dicke von im Wesentlichen 0,0102 mm (0,4 mil) bis im Wesentlichen 0,038 mm (1,5 mil) aufweist.

6. Gegenstand gemäß Anspruch 1, wobei die hydrophobe Schicht (20) ausgewählt ist aus der Gruppe bestehend aus atmungsaktiven Olefinen, elastomeren Copolyestern, Urethanen und Kombinationen davon.

7. Gegenstand gemäß Anspruch 1, wobei die hydrophobe Schicht (20) ein Polyesterurethan umfasst.

8. Gegenstand gemäß Anspruch 1, wobei die hydrophobe Schicht (20) eine Wasserdampf-Übergangsrate von im Wesentlichen 250 g/m²/24-Stunden bis im Wesentlichen 1000 g/m²/24-Stunden aufweist.

9. Gegenstand gemäß Anspruch 8, wobei die hydrophobe Schicht (20) eine Dicke von im Wesentlichen 0,0102 mm (0,4 mil) bis im Wesentlichen 0,127 mm (5 mil) aufweist.

10. Gegenstand gemäß Anspruch 1, wobei die hautseitige Seite der hydrophilen Schicht (10) und die hydrophobe Schicht (20) oder beide einen Überzug auf wenigstens einem Teil davon aufweist/aufweisen, umfassend einen medizinisch anerkannten Klebstoff, ausgewählt aus der Gruppe bestehend aus Acrylen, Hydrokolloiden, Hydrogelen, Polyurethanen, Siliconen und Kombinationen davon.

11. Gegenstand gemäß Anspruch 1, wobei der Umfang der hydrophoben Schicht (20) über den Umfang der hydrophilen Schicht (10) um einen Anstand von im Wesentlichen 0,794 mm (1/32 Inch) bis im Wesentlichen 19,05 mm (3/4 Inch) hinausragt.

12. Gegenstand gemäß Anspruch 1, wobei die Abgabeschicht (30) Olefine, Polyester, Copolymere und Kombinationen davon umfasst.

13. Gegenstand gemäß Anspruch 1, wobei die Abgabeschicht (30) ein Polyethylen/Ethylenvinylacetat-Gemisch umfasst.

14. Gegenstand gemäß Anspruch 1, wobei die Abgabeschicht (30) ein Fenster (32) aufweist, das durch Entfernen eines mittleren Teils der Abgabeschicht darin gebildet ist.

15. Gegenstand gemäß Anspruch 1, ferner umfassend ein Klebeband (40) zwischen der hydrophoben Schicht (20) und der Abgabeschicht (30).

16. Gegenstand gemäß Anspruch 1, ferner umfassend eine Stabilisatorschicht (60) zwischen der hydrophilen Schicht (10) und der hydrophoben Schicht (20).

17. Gegenstand gemäß Anspruch 16, wobei die Stabilisatorschicht (60) aus einem Material gebildet ist, ausgewählt aus der Gruppe bestehend aus Gaze, Stoff, thermisch verfestigtem Polypropylen, Polypropylen-Spinnvlies, Nylon, wasservernadeltem Polyester, Celluloseacetat-Vlies, gewobenem Acetat-Taft.

18. Gegenstand gemäß Anspruch 1, ferner umfassend ein absorbierendes Material (70) an der hautseitigen Seite der hydrophilen Schicht (10).

19. Gegenstand gemäß Anspruch 18, wobei das absorbierende Material (70) ausgewählt ist aus der Gruppe bestehend aus Baumwolle, Alginat, Rayon, Cellulose, Urethanen, Hydrogelen, Hydrokolloiden, Polyethylenoxiden, superabsorbierenden Polymeren und Kombinationen davon.

20. Gegenstand gemäß Anspruch 1, wobei die hydrophile Schicht (10), die hydrophobe Schicht (20) und die Abgabeschicht (30) Aussparungen (14, 24, 34) aufweisen, die miteinander fluchten.

21. Gegenstand gemäß Anspruch 1, wobei der Gegenstand eine Dicke von im Wesentlichen 0,076 mm (3 mil) bis im Wesentlichen 0,279 mm (11 mil) aufweist.

22. Gegenstand gemäß Anspruch 1, ferner umfassend ein medizinisches Mittel, ausgewählt aus der Gruppe bestehend aus antimikrobiellen Mitteln, Analgetika, Antipyretika, Anästhetika, Antiepileptika, Antihistaminika, entzündungshemmenden Mitteln, diagnostischen Mitteln, Sympathomimetika, Parasympathomimetika, Cholinomimetika, Antimuskarinika, Antispasmodika, Hormonen, Hormonanaloga, Wachstumsfaktoren, Muskelrelaxantien, Antineoplastika, Immunsuppressiva, Steroiden, Polysacchariden, Enzymen, Beruhigungsmitteln, Sulfonamiden, Impfstoffen, Vitaminen, Antimalariamitteln, Antimigränemitteln, Anti-Parkinson-Mitteln, Anticholinergika, Herz-Kreislauf-Mitteln, Alkaloiden, Narkotika, Opioidrezeptor-Antagonisten, Antikrebsmitteln, Antikonvulsiva, Antiemetika, Antihistaminika, Prostaglandinen, zytotoxischen Arzneimitteln, Estrogenen, antibakteriellen Mitteln, fungiziden Mitteln, antiviralen Mitteln, Antikoagulantien, Antikonvulsiva, Antidepressiva, immunologischen Mitteln, Antigenen, Blutkoagulationsfaktoren, Proteinhemmern, Proteinantagonisten, Proteinagonisten, Nucleinsäuren, Oligonucleotiden, Ribozymen und Kombinationen davon.

23. Gegenstand gemäß Anspruch 1, wobei
die hydrophile Schicht (10) eine Wasserdampf-Übergangsrate von im Wesentlichen 400 g/m²/24-Stunden bis im Wesentlichen 3000 g/m²/24-Stunden aufweist und eine hautseitige Seite und eine dazu gegenüberliegende Seite aufweist;
die hydrophobe Schicht (20), die der hydrophilen Schicht an der Seite, die der hautseitigen Seite gegenüberliegt, benachbart ist, eine Wasserdampf-Übergangsrate von im Wesentlichen 250 g/m²/24-Stunden bis im Wesentlichen 1000 g/m²/24-Stunden aufweist und ein Fenster (22) aufweist, das durch Entfernen eines mittleren Teils der hydrophoben Schicht darin gebildet ist;
die Abgabeschicht (30), die der hydrophoben Schicht an der Seite, die der hydrophilen Schicht gegenüberliegt, benachbart ist, ein Fenster (32) aufweist, das durch Entfernen eines mittleren Teils der Abgabeschicht darin gebildet ist; und
wobei die Fenster in der hydrophoben Schicht und der Abgabeschicht miteinander fluchten und der Gegenstand eine Dicke von im Wesentlichen 0,076 mm (3 mil) bis im Wesentlichen 0,279 mm (11 mil) aufweist.

24. Gegenstand gemäß Anspruch 23, wobei sich die Abziehschicht (50) an der Seite der hydrophilen Schicht (10), die der hydrophoben Schicht (20) gegenüberliegt, befindet.

## Revendications

1. Article comprenant :
au moins une couche hydrophile (10) ayant un côté tourné vers la peau et un côté opposé à la peau ;
au moins une couche hydrophobe (20) adjacente à la couche hydrophile du côté opposé au côté tourné vers la peau ;
une couche de distribution (30) adjacente à la couche hydrophobe du côté opposé à la couche hydrophile ; et
une couche de séparation (50) du côté de la couche hydrophile opposé à la couche hydrophobe,
dans lequel la couche hydrophobe renferme une fenêtre (22) formée par élimination d'une portion centrale de la couche hydrophobe.

2. Article suivant la revendication 1, dans lequel la couche hydrophile (10) est formée d'un polymère choisi dans le groupe consistant en l'alcool polyvinylique réticulé, la polyvinylpyrrolidone réticulée, des polyuréthannes hydrophiles, des esters hydroxyalkyliques hydrophiles d'acide poly(méth)acrylique et leurs copolymères, des polymères polyéther-polyamide hydrophiles, des dérivés de cellulose hydrophiles et insolubles dans l'eau et leurs associations.

3. Article suivant la revendication 1, dans lequel la couche hydrophile (10) comprend un polyuréthanne hydrophile formé de polyéthylèneglycol, de polypropylèneglycol, ou de leurs associations avec un diisocyanate et, éventuellement, un éthanediol ou l'éthylènediamine.

4. Article suivant la revendication 1, dans lequel la couche hydrophile (10) a une vitesse de transition de vapeur d'eau de substantiellement 400 g/m²/24 heures à substantiellement 3000 g/m²/24 heures.

5. Article suivant la revendication 4, dans lequel la couche hydrophile (10) a une épaisseur allant de substantiellement 0,0102 mm (0,4 mil) à substantiellement 0,038 mm (1,5 mil).

6. Article suivant la revendication 1, dans lequel la couche hydrophobe (20) est choisie dans le groupe consistant en des oléfines perméables, des copolyesters élastomères, des uréthannes et leurs associations.

7. Article suivant la revendication 1, dans lequel la couche hydrophobe (20) comprend un polyester-uréthanne.

8. Article suivant la revendication 1, dans lequel la couche hydrophobe (20) a une vitesse de transition de vapeur d'eau allant de substantiellement 250 g/m²/24 heures à substantiellement 1000 g/m²/24 heures.

9. Article suivant la revendication 8, dans lequel la couche hydrophobe (20) a une épaisseur allant de substantiellement 0,0102 mm (0,4 mil) à substantiellement 0,127 mm (5 mil).

10. Article suivant la revendication 1, dans lequel le côté tourné vers la peau de la couche hydrophile (10) et/ou la couche hydrophobe (20) comporte(nt) un revêtement sur au moins une de leur partie comprenant un adhésif admis du point de vue médical choisi dans le groupe consistant en des résines acryliques, des hydrocolloïdes, des hydrogels, des polyuréthannes, des silicones et leurs associations.

11. Article suivant la revendication 1, dans lequel le périmètre de la couche hydrophobe (20) s'étend au-delà du périmètre de la couche hydrophile (10) d'une distance de substantiellement 0,794 mm (1/32 inch) à substantiellement 19,05 mm (3/4 inch).

12. Article suivant la revendication 1, dans lequel la couche d'administration (30) comprend des oléfines, des polyesters, leurs copolymères et leurs associations.

13. Article suivant la revendication 1, dans lequel la couche d'administration (30) comprend un mélange polyéthylène / éthylène-acétate de vinyle.

14. Article suivant la revendication 1, dans lequel la couche d'administration (30) renferme une fenêtre (32) formée par élimination d'une portion centrale de la couche d'administration.

15. Article suivant la revendication 1, comprenant en outre un ruban adhésif (40) entre la couche hydrophobe (20) et la couche d'administration (30).

16. Article suivant la revendication 1, comprenant en outre une couche de stabilisant (60) entre la couche hydrophile (10) et la couche hydrophobe (20).

17. Article suivant la revendication 16, dans lequel la couche de stabilisant (60) est formée d'une matière choisie dans le groupe consistant en une gaze, une étoffe, un polypropylène thermiquement lié, un polypropylène non tissé, un Nylon, un polyester hydro-enchevêtré, un acétate cellulosique non tissé et un acétate sous forme de taffetas tissé.

18. Article suivant la revendication 1, comprenant en outre une matière absorbante (70) sur le côté tourné vers la peau de la couche hydrophile (10).

19. Article suivant la revendication 18, dans lequel la matière absorbante (70) est choisie dans le groupe consistant en le coton, un alginate, la rayonne, la cellulose, des uréthannes, des hydrogels, des hydrocolloïdes, des poly(oxydes d'éthylène), des polymères hyperabsorbants et leurs associations.

20. Article suivant la revendication 1, dans lequel la couche hydrophile (10), la couche hydrophobe (20) et la couche d'administration (30) renferment des entailles (14, 24, 34) qui sont mutuellement contiguës.

21. Article suivant la revendication 1, ledit article ayant une épaisseur allant de substantiellement 0,076 mm (3 mil) à substantiellement 0,279 mm (11 mil).

22. Article suivant la revendication 1, comprenant en outre un agent médicamenteux choisi dans le groupe consistant en des agents antimicrobiens, des analgésiques, des antipyrétiques, des anesthésiques, des anti-épileptiques, des antihistaminiques, des anti-inflammatoires, des agents de diagnostic, des agents sympathomimétiques, des agents parasympathomimétiques, des agents cholinomimétiques, des agents antimuscariniques, des antispasmodiques, des hormones, des analogues d'hormones, des facteurs de croissance, des myorelaxants, des anti-néoplasiques, des immunosuppresseurs, des stéroïdes, des polysaccharides, des enzymes, des tranquillisants, des sulfonamides, des vaccins, des vitamines, des agents antipaludiques, des agents antimigraineux, des agents anti-parkinsoniens, des anticholinergiques, des agents cardio-vasculaires, des alcaloïdes, des narcotiques, des antagonistes des récepteurs d'opioïdes, des agents anticancéreux, des anticonvulsivants, des antiémétiques, des antihistaminiques, des prostaglandines, des médicaments cytotoxiques, des oestrogènes, des agents antibactériens, des antifongiques, des antiviraux, des anticoagulants, des anticonvulsivants, des antidépresseurs, des agents immunologiques, des antigènes, des facteurs de coagulation sanguine, des inhibiteurs de protéines, des antagonistes des protéines, des agonistes de protéines, des acides nucléiques, des oligonucléotides, des ribozymes, et leurs associations.

23. Article suivant la revendication 1, dans lequel :
la couche hydrophile (10) possède une vitesse de transition de vapeur d'eau allant de substantiellement 400 g/m²/24 heures à substantiellement 3 mg/m²/24 heures et comporte un côté tourné vers la peau, et un côté opposé à celui-ci ;
la couche hydrophobe (20) adjacente à la couche hydrophile du côté opposé au côté tourné vers la peau possède une vitesse de transition de vapeur d'eau allant de substantiellement 250 g/m²/24 heures à substantiellement 1000 g/m²/24 heures et renferme une fenêtre (22) formée par élimination d'une portion centrale de la couche hydrophobe ;
la couche d'administration (30) adjacente à la couche hydrophobe du côté opposé à la couche hydrophile renferme une fenêtre (32) formée par élimination d'une portion centrale de la couche d'administration ; et
dans lequel les fenêtres dans la couche hydrophobe et la couche d'administration sont mutuellement contiguës, l'article ayant une épaisseur allant de substantiellement 0,076 mm (3 mil) à substantiellement 0,279 mm (11 mil).

24. Article suivant la revendication 23, dans lequel la couche de séparation (50) est du côté de la couche hydrophile (10) opposé à la couche hydrophobe (20).
